(19) Europäisches Patentamt / European Patent Office / Office européen des brevets

(11) **EP 4 631 536 A1**

(12) **EUROPEAN PATENT APPLICATION**
published in accordance with Art. 153(4) EPC

(43) Date of publication:
**15.10.2025 Bulletin 2025/42**

(21) Application number: **23901026.7**

(22) Date of filing: **04.12.2023**

(51) International Patent Classification (IPC):
***A61L 27/20** (2006.01)* ***A61L 27/52** (2006.01)*
***A61L 27/54** (2006.01)*

(52) Cooperative Patent Classification (CPC):
**A61L 27/20; A61L 27/52; A61L 27/54**

(86) International application number:
**PCT/KR2023/019790**

(87) International publication number:
**WO 2024/123007 (13.06.2024 Gazette 2024/24)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **05.12.2022 KR 20220168082**

(71) Applicant: **Medytox Inc.**
**Chungcheongbuk-do 28126 (KR)**

(72) Inventors:
- **LIM, Cheon Soo**
  **Sejong 30092 (KR)**
- **KIM, Se Na**
  **Sejong 30141 (KR)**
- **RHEE, Chang Hoon**
  **Gunpo-si, Gyeonggi-do 15823 (KR)**

(74) Representative: **Brevalex**
**Tour Trinity**
**1 B Place de la Défense**
**92400 Courbevoie (FR)**

(54) **CROSS-LINKED HYALURONIC ACID GEL HAVING GOOD SPREADABILITY AND STABILITY, AND USE THEREOF**

(57) Provided are a cross-linked hyaluronic acid having good spreadability and stability, and uses thereof.

FIG. 1

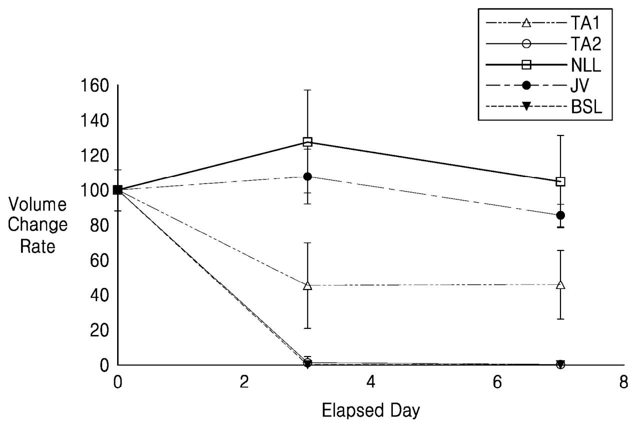




EP 4 631 536 A1

## Description

### Technical Field

The present invention relates to a cross-linked hyaluronic acid gel having good spreadability and stability, and uses thereof.

### Background Art

Hyaluronic acid is a natural component of the dermis. Hyaluronic acid plays an important role in hydration and elasticity of the skin. As the skin ages, both the quantity and quality of hyaluronic acid in the skin decreases. These changes cause skin dryness and formation of wrinkles.

Hyaluronic acid may be used for hydration, wrinkles, or wound healing. However, hyaluronic acid may be degraded in the body by factors such as enzymes, temperature, and free radicals. Therefore, the lifespan of hyaluronic acid in the body is limited.

Therefore, when hyaluronic acid is used for hydration, there is a need for a hyaluronic acid gel that is capable of being retained in the body for a long period of time, has good spreadability, and does not cause lump formation.

### Disclosure

### Technical Problem

An aspect provides a cross-linked hyaluronic acid gel having good spreadability and stability upon in vivo injection, wherein the cross-linked hyaluronic acid gel has an elastic modulus (G') of 0.01 Pa to 20 Pa, a viscous modulus (G") of 0.1 Pa to 20 Pa, and an elasticity percentage (elasticity %) of 10 % to 40 %. The cross-linked hyaluronic acid gel may have an average particle diameter of 35 $\mu$m to 100 $\mu$m.

Another aspect provides a composition for tissue filling or skin hydration including the cross-linked hyaluronic acid gel.

Another aspect provides a method of filling tissue of a subject or hydrating skin of a subject, including administering to the subject the composition for tissue filling or skin hydration including the cross-linked hyaluronic acid gel.

### Technical Solution

An aspect provides a cross-linked hyaluronic acid gel having good spreadability and stability upon in vivo injection, wherein the cross-linked hyaluronic acid gel has an elastic modulus (G') of 0.01 Pa to 20 Pa, a viscous modulus (G") of 0.1 Pa to 20 Pa, and an elasticity percentage of 10% to 40%. The cross-linked hyaluronic acid gel may have an average particle diameter of 35 $\mu$m to 100 $\mu$m.

As used herein, the term "cross-linked hyaluronic acid gel" refers to a composition containing an injectable cross-linked hyaluronic acid. The composition may be an injectable dermal filler composition. The term "gel" as used herein generally refers to a material having a fluidity between that of a liquid and a solid at room temperature. Additionally, the term "gel" refers to a material capable of absorbing water (i.e., a hydrogel). The composition may generally include a nonpyrogenic isotonic buffer, such as a physiologically acceptable carrier fluid, for example, buffered physiological saline.

The gel may contain 1.5 wt% to 3.0 wt% of cross-linked hyaluronic acid based on the weight of the gel composition.

The term "injectable" indicates that the composition is suitable for being injected into the skin or other tissue to deliver the composition to the desired target site.

An "injectable" composition may be administered through a syringe under normal conditions and pressure.

As used herein, the term "hyaluronic acid" refers to hyaluronan, hyaluronate, or a pharmaceutically acceptable salt thereof having a chemical structure of Formula 1.

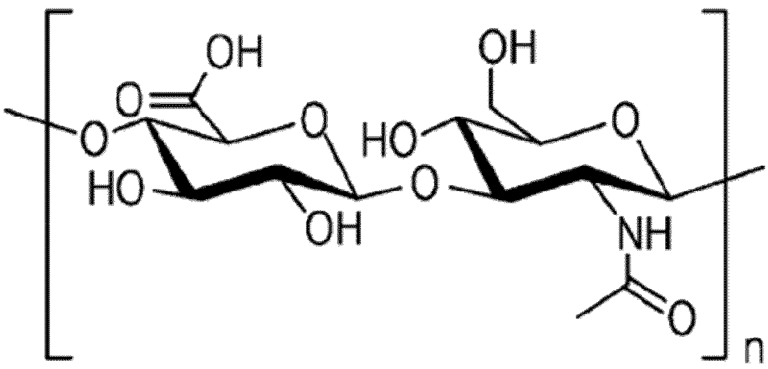

(1)

In Formula 1, n is the number of repeating units. Hyaluronic acid from all origins, including bacterial and algal origin, is useful.

The term "cross-linked" as used herein refers to two or more polymer chains of hyaluronic acid that are covalently linked via a cross-linking agent. Such cross-linking may be distinguished by intermolecular or intramolecular dehydration giving rise to lactones, anhydrides, or esters within a single polymer or within two or more chains. Intramolecular cross-linking may also be included in the compositions described herein.

The term "cross-linking agent" contains at least two reactive functional groups that form a covalent bond between two or more molecules. The cross-linking agent may be homodifunctional or heterobifunctional. The cross-linking agent as used herein may be one that contains a functional group complementary to the functional group of hyaluronic acid, thereby allowing a cross-linking reaction to proceed.

The term "elasticity percentage (elasticity %)" is defined as "elastic modulus (G')/ (elastic modulus (G') + viscous modulus (G")) x100". A material may have a similar elasticity percent regardless of whether its viscous modulus is high or low. The elasticity percentage indicates the directionality toward hardness or softness of a cross-linked hyaluronic acid gel. The lower the elasticity percentage, the softer and more spreadable the cross-linked hyaluronic acid gel is.

The phrase "spreadability" and grammatical variations thereof refer to the degree to which a material initially spreads into surrounding tissues after injection, without clumping, swelling, lifting, or lump formation at the injection site. Additionally, "good spreadability" and grammatical variations thereof may refer to that the percentage ratio (%) of the volume to the initial volume at the injection site at the early stage of injection is 100 % or less. For example, in the present invention, the volume ratio of the material between day 0 and day 3, between day 0 and day 2, or between day 0 and day 1 after injection (day 0) may be in the range of 100 % to 90 %, 100 % to 80 %, 100 % to 60 %, 100 % to 50 %, 100 % to 30 %, 100 % to 20 %, 100 % to 10 %, 100 % to 0 %, 90 % to 80 %, 90 % to 60 %, 90 % to 50 %, 90 % to 30 %, 90 % to 20 %, 90 % to 10 %, 90 % to 0 %, 80 % to 60 %, 80 % to 50 %, 80 % to 30 %, 80 % to 20 %, 80 % to 10 %, or 80 % to 0 %, relative to the initial injection volume. The volume ratio is calculated by the following formula:

$$\text{Volume ratio (\%)} = \text{Volume after injection} / \text{Volume at initial injection} \times 100$$

The measurement site for the volume ratio is within a range of 0.5 cm to 1 cm in radius centered on the injection site.

The term "Degradation 50" refers to the time required for the elastic modulus (G') to decrease to 50 % of its initial measured value as hyaluronic acid is degraded following treatment with a degrading enzyme such as hyaluronidase in vitro. It is known that the longer the Degradation 50, the higher the degradation resistance, indicating that the hyaluronic acid may stably remain in the body for a longer period. High degradation resistance of hyaluronic acid indicates that the hyaluronic acid exhibits high in vivo stability.

The cross-linked hyaluronic acid gel has an elastic modulus (G') of 0.01 Pa to 20 Pa, for example, 0.01 Pa to 15 Pa, 0.01 Pa to 10 Pa, 0.01 Pa to 5 Pa, 0.01 Pa to 4.0 Pa, 0.01 Pa to 3.5 Pa, 0.01 Pa to 3.0 Pa, 0.01 Pa to 2.5 Pa, 0.01 Pa to 2.0 Pa, 0.01 Pa to 1.5 Pa, 0.01 Pa to 1.0 Pa, 0.1 Pa to 20 Pa, 0.1 Pa to 15 Pa, 0.1 Pa to 10 Pa, 0.1 Pa to 5 Pa, 0.1 Pa to 4 Pa, 0.1 Pa to 3.5 Pa, 0.1 Pa to 3.0 Pa, 0.1 Pa to 2.5 Pa, 0.1 Pa to 2.0 Pa, 0.1 Pa to 1.5 Pa, 0.1 Pa to 1.0 Pa, 0.05 Pa to 5 Pa, 0.05 Pa to 4.0 Pa, or 0.105 Pa to 1.035 Pa.

The cross-linked hyaluronic acid gel may have a viscous modulus (G") of 0.1 Pa to 20 Pa, for example, 0.1 Pa to 15 Pa, 0.1 Pa to 10 Pa, 0.1 Pa to 5 Pa, 0.1 Pa to 4 Pa, 0.1 Pa to 3.5 Pa, 0.1 Pa to 3.0 Pa, 0.1 Pa to 2.5 Pa, 0.1 Pa to 2.0 Pa, 0.1 Pa to 1.5 Pa, 0.1 Pa to 1.0 Pa, 0.5 Pa to 1.0 Pa, 0.6 Pa to 1.0 Pa, or 0.7 Pa to 1.0 Pa.

The cross-linked hyaluronic acid gel may have an elasticity percentage (elasticity %) of 10 % to 40 %, for example, 10 % to 30 %, 20 % to 30 %, 20 % to 27 %, 20 % to 25 %, 21 % to 25 %, or 22 % to 24 %.

The cross-linked hyaluronic acid gel may have an average particle diameter of 35 μm to 100 μm, for example, 35 μm to 90 μm, 35 μm to 80 μm, 35 μm to 70 μm, 35 μm to 60 μm, 35 μm to 65 μm, 35 μm to 50 μm, 35 μm to 45 μm, 40 μm to 65 μm, 40 μm to 60 μm, 40 μm to 55 μm, 40 μm to 50 μm, 45 μm to 50 μm, 40 μm to 45 μm, or 41 μm to 44 μm.

The cross-linked hyaluronic acid gel has a Degradation 50 of 90 minutes to 300 minutes, for example, 90 minutes to 290 minutes, 90 minutes to 280 minutes, 90 minutes to 270 minutes, 90 minutes to 260 minutes, 90 minutes to 250 minutes, 90 minutes to 245 minutes, 100 minutes to 250 minutes, 100 minutes to 245 minutes, 120 minutes to 350 minutes, 130 minutes to 350 minutes, 140 minutes to 300 minutes, 150 minutes to 300 minutes, 200 minutes to 300 minutes, 200 minutes to 290 minutes, 200 minutes to 280 minutes, 200 minutes to 270 minutes, 200 minutes to 260 minutes, 200 minutes to 145 minutes, 210 minutes to 300 minutes, 210 minutes to 290 minutes, 210 minutes to 280 minutes, 210 minutes to 270 minutes, 210 minutes to 260 minutes, 220 minutes to 300 minutes, 230 minutes to 260 minutes, 235 minutes to 260 minutes, 235 minutes to 250 minutes, or 240 minutes to 260 minutes.

The cross-linked hyaluronic acid gel may be cross-linked with a cross-linking agent having a bifunctional epoxy group. The cross-linking agent having a bifunctional epoxy group may be 1,4-butanediol diglycidyl ether (BDDE), ethylene glycol diglycidyl ether (EGDGE), 1-ethyl-3-(3-dimethylaminopropyl)carbodiimide (EDC), biscarbodiimide (BCDI), diglycerol polyglycidyl ether, divinylsulfone (DVS), poly(ethylene glycol) diglycidyl ether (PEGDE), poly(propylene glycol) diglycidyl ether (PPGDE), poly(tetramethylene glycol) diglycidyl ether, polyglycerol polyglycidyl ether, glycerol diglycidyl ether, triethylene diglycidyl ether, trimethylolpropane triglycidyl ether, ethylene diglycidyl ether, neopentyl glycol diglycidyl ether, 1,6-hexanediol diglycidyl ether, or a combination thereof.

The cross-linked hyaluronic acid gel may take the form of a gel in a solution, for example, in an aqueous solution. In this case, the term "hyaluronic acid" is used interchangeably with "hyaluronic acid gel".

In the cross-linked hyaluronic acid gel, the hyaluronic acid prior to cross-linking may be derived from any origin. The hyaluronic acid prior to cross-linking may be derived from, for example, a non-animal source. The hyaluronic acid prior to cross-linking may be derived from bacteria. The bacteria may be derived from the genus *Streptococcus.* The *Streptococcus* genus bacteria may be *Streptococcus equi, S. pyogenes or S. zooepidemicus.* The hyaluronic acid prior to cross-linking may also be commercially available. The intrinsic viscosity of the hyaluronic acid prior to cross-linking may be from 1.0 $m^3$/kg to 4.0 $m^3$/kg.

The cross-linked hyaluronic acid gel may be prepared by a method including incubating a reaction mixture containing a cross-linking agent and hyaluronic acid to proceed a cross-linking reaction. The incubation may be incubation while heating. The heating may be heating to a temperature of 30 °C to 60 °C, 30 °C to 50 °C, or 35 °C to 45 °C. The incubation may be performed for 10 hours to 40 hours, 10 hours to 30 hours, 10 hours to 25 hours, or 15 hours to 25 hours. The cross-linking agent may have a multifunctional group. The cross-linking agent may have a bifunctional epoxy group as described above.

The cross-linked hyaluronic acid gel may have a cross-link rate of 0.5 % to 7 %. The cross-link rate may be, for example, 0.6 % to 7 %, 0.7 % to 7 %, 0.8 % to 7 %, 0.9 % to 7 %, 1.0 % to 7 %, 0.5 % to 5 %, 0.5 % to 3.0 %, 0.5 % to 2.0 %, 1.0 % to 5 %, 1.0 % to 3.0 %, 1.0% to 2.5 %, or 1.0 % to 2.0 %.

As used herein, the term "cross-link rate (%)" refers to the ratio of covalently bonded cross-linking agent per 100 disaccharide repeating units of hyaluronic acid. The disaccharide repeating unit is composed of D-glucuronic acid and N-acetylglucosamine. The cross-link rate may be determined by known methods. For example, it may be determined by ion exchange chromatography (IEC) or NMR. Ion exchange chromatography is a method that performs reversible ion exchange between a stationary phase and a mobile phase, and separates and analyzes sample ions based on their affinity differences for the stationary phase. When using IEC, the cross-link rate may be calculated by the following formula:

$$\text{Cross-link rate (\%)} = [\Sigma(\text{Peak area} \times \text{Number of hyaluronic acid disaccharide repeating units} \times \text{Ratio of cross-linked disaccharide repeating units}) \times 100]/[\Sigma(\text{Peak area} \times \text{Number of hyaluronic acid disaccharide repeating units})]$$

The cross-linked hyaluronic acid gel may be more stable in the body compared to natural hyaluronic acid or some cross-linked hyaluronic acid gels known in the art. The cross-linked hyaluronic acid gel may be a more stable hyaluronic acid gel compared to, for example, Belotero soft lidocaine (Merz Aesthetics). The cross-linked hyaluronic acid gel may be more stable in the body than a composition including natural hyaluronic acid or, for example, than an administration of Belotero soft lidocaine.

The cross-linked hyaluronic acid gel may remain stable in the body for 28 days or more, 29 days or more, 30 days or more, 31 days or more, 32 days or more, 33 days or more, 34 days or more, 35 days or more, 36 days or more, 37 days or more, 38 days or more, 39 days or more, 40 days or more, 41 days or more, 42 days or more, 43 days or more, 44 days or more, 45 days or more, 46 days or more, 47 days or more, 48 days or more, 49 days or more, 50 days or more, 51 days or more, 52 days or more, 53 days or more, 54 days or more, 55 days or more, 56 days or more, 57 days or more, 58 days or more, 59 days or more, 60 days or more, 61 days or more, 62 days or more, 63 days or more, 64 days or more, 65 days or more, 66 days or more, 67 days or more, 68 days or more, 69 days or more, 70 days or more, 71 days or more, 72 days or

more, 73 days or more, 74 days or more, 75 days or more, 76 days or more, 77 days or more, 78 days or more, 79 days or more, 80 days or more, 81 days or more, 82 days or more, 83 days or more, 84 days or more, 85 days or more, 86 days or more, 87 days or more, 88 days or more, 89 days or more, 90 days or more, 91 days or more, 92 days or more, 93 days or more, 94 days or more, 95 days or more, 96 days or more, 97 days or more, 98 days or more, 99 days or more, 100 days or more, 101 days or more, 102 days or more, 103 days or more, 104 days or more, 105 days or more, 106 days or more, 107 days or more, 108 days or more, 109 days or more, 110 days or more, 111 days or more, 112 days or more, 120 days or more, 130 days or more, 140 days or more, 150 days or more, 160 days or more, 170 days or more, 180 days or more, 28 days to 84 days, 29 days to 84 days, 30 days to 84 days, 35 days to 84 days, 40 days to 84 days, 45 days to 84 days, 50 days to 84 days, 54 days to 84 days, 55 days to 84 days, 60 days to 84 days, 28 days to 112 days, 29 days to 112 days, 30 days to 112 days, 35 days to 112 days, 40 days to 112 days, 45 days to 112 days, 50 days to 112 days, 54 days to 112 days, 55 days to 112 days, 60 days to 112 days, 70 days to 112 days, 80 days to 112 days, 90 days to 112 days, 100 days to 112 days, 105 days to 112 days, 28 days to 180 days, 56 days to 180 days, 56 days to 180 days, or 112 days to 180 days.

The term "lump" phenomenon as used herein refers to a unnatural condition in which the skin surface at or around the injection site becomes unevenly raised due to the injected composition, resulting in an appearance that is unnatural compared to the surrounding area. In general, the lower the viscoelasticity value or the lower the elasticity percentage, the better the spreadability within the tissue into the surrounding area, which tends to reduce the occurrence of lump formation.

Another aspect provides a composition for tissue filling or skin hydration including the cross-linked hyaluronic acid gel described above. In the composition, the cross-linked hyaluronic acid gel may have the physical properties described above, such as elastic modulus, viscous modulus, elasticity percentage, average particle diameter, cross-link rate, and Degradation 50.

In the composition, the concentration of the cross-linked hyaluronic acid gel may be 10 mg/ml or more, 15 mg/ml or more, 18 mg/ml or more, or 19 mg/ml or more. For example, the concentration of the cross-linked hyaluronic acid gel may be 10 mg/ml to 30 mg/ml, 12 mg/ml to 28 mg/ml, 14 mg/ml to 26 mg/ml, 15 mg/ml to 25 mg/ml, 16 mg/ml to 24 mg/ml, 18 mg/ml to 22 mg/ml, or about 20 mg/ml.

The composition may be used for tissue repair or hydration in a subject.

In the composition, the subject may be a mammal. The mammal may be a human, a dog, a cat, a cow, a pig, a rat, or a sheep.

The composition may not additionally include non-cross-linked hyaluronic acid. "Additionally including" non-cross-linked hyaluronic acid means that non-cross-linked hyaluronic acid is artificially added to the cross-linked hyaluronic acid gel. Additionally, "not additionally including non-cross-linked hyaluronic acid" means that non-cross-linked hyaluronic acid is not artificially added to the cross-linked hyaluronic acid gel.

The composition may further include an anesthetic. The anesthetic may be a local anesthetic. The local anesthetic may be, but is not limited to, at least one of ambucaine, amolanone, amylocaine, benoxinate, benzocaine, betoxycaine, biphenamine, bupivacaine, butacaine, butamben, butanilicaine, butethamine, butoxycaine, carticaine, chloroprocaine, cocaethylene, cocaine, cyclomethycaine, dibucaine, dimethysoquin, dimethocaine, diperodon, dicyclomine, ecgonidine, ecgonine, ethyl chloride, etidocaine, beta-eucaine, euprocin, phenalcomine, formocaine, hexylcaine, hydroxytetracaine, isobutyl paminobenzoate, leucinocaine mesylate, levoxadrol, lidocaine, mepivacaine, meprylcaine, metabutoxycaine, methyl chloride, myrtecaine, naepaine, octacaine, orthocaine, oxethazaine, parethoxycaine, phenacaine, phenol, piperocaine, piridocaine, polidocanol, pramoxine, prilocaine, procaine, propanocaine, proparacaine, propiocaine, propoxycaine, pseudococaine, pyrrocaine, ropivacaine, salicyl alcohol, tetracaine, tolycaine, trimecaine, zolamine, or a salt thereof.

The composition may further include a pharmacologically active material or a material having a biological function. The material may be a functional polymer material. The functional polymer material may be a natural polymer or a synthetic polymer. The pharmacologically active material or a material having a biological function may be, for example, at least one material selected from the group consisting of collagen, chitosan, alginate, gelatin, polynucleotide, polydeoxyribonucleotide, silk fibroin, elastin, tropoelastin, carrageenan, gamma-polyglutamic acid, sulfated hyaluronic acid, chondroitin sulfate, fucoidan, heparin, heparan sulfate, pentosan polysulfate, pullulan, and keratin.

The collagen may be at least one material selected from the group consisting of bovine-derived, porcine-derived, fish-derived, human recombinant-derived, human recombinant collagen peptide, atelocollagen, human recombinant collagen peptide alpha-1 chain, human recombinant collagen peptide derivative, RGD sequence-enhanced human recombinant collagen peptide, and RGD sequence-enhanced human recombinant collagen peptide alpha-1 chain.

The collagen may be selected from the group consisting of collagen types I, II, III, IV, V, VI, VII, VIII, IX, X, XI, XII, XIII, XIV, XVII, XVIII, XIX, XX, XXI, XXII, XXIII, XXIV, XXV, XXVI, XXVII, and XXVIII.

The pharmacologically active material or a material having a biological function may be at least one material selected from the group consisting of polyethylene glycol, polylactic acid, polyglycolic acid, polylactic-co-glycolic acid, polycaprolactone, poloxamer, polyphosphazene, and polydioxanone.

The composition may be filled in a syringe.

The composition may be intended for use in at least one application selected from the group consisting of skin

moisturization, facial plastic surgery, wrinkle improvement, facial contouring, breast plastic surgery, urinary incontinence treatment, arthritis treatment and drug delivery.

In the composition, when 1 ml of the pre-filled composition in a syringe is injected at a rate of 12 mm/min using a 22 gauge (G) to 31 gauge (G), 10 mm to 13 mm needle, the injection force may be 50 N or less. In the composition, when 1 ml of the pre-filled composition in a syringe is injected at a rate of 12 mm/min using a 27 gauge (G), 13 mm needle, the injection force may be 50 N or less.

The composition may further include a pharmaceutically acceptable carrier, excipient and diluent. The carrier may include, for example, water or a buffer. The buffer may be such that the pH of the solution hardly changes with the addition of the components of the composition. The composition may be an aqueous liquid composition. The composition may be an aqueous buffered composition. The pH of the aqueous buffered composition may be within the physiological pH range, for example, between about 6.0 and 8.0, for example, from about 6.0 to about 7.5, from about 6.5 to about 7.5, or from about 6.8 to about 7.2. The pH may be adjusted by the addition of an appropriate acid or base, such as HCl, $Na_2CO_3$ or NaOH. In an embodiment, the aqueous buffered composition may include phosphate buffered saline (PBS). In another embodiment, the aqueous buffered composition may include Tris(hydroxymethyl)aminomethane. In certain embodiments, additional solutes, such as sodium chloride, calcium chloride, and potassium chloride, may be added to adjust osmolarity and ion concentration.

The composition may be a composition in which the cross-linked hyaluronic acid gel is suspended in an aqueous solvent. The aqueous solvent may be a buffer such as PBS, water or saline. The composition may be sterilized.

The composition may be contained in a container. The container may be a syringe. The composition may be pre-filled into a syringe before use. The composition may be administered using a pre-filled syringe.

Additionally, the composition may be used for administration into the skin for hydration within tissue. As used herein, the term "hydration" may refer to increasing or maintaining moisture in tissue. Such increase or maintenance includes supplying moisture to tissue such as skin by injecting hyaluronic acid and cross-linked hyaluronic acid gel, which inherently have high water content, or replenishing moisture at the injection site by drawing water from the surrounding tissue due to the highly hydrophilic nature of hyaluronic acid. Therefore, the hydration involves supplying and retaining moisture in the tissues. In certain embodiments, the term "hydration" may be used interchangeably with "moisturizing." The composition may be used for administration into the skin for hydration. The composition may be used for administration into wrinkle-free skin for hydration.

Another aspect provides a device including the composition described above. The device may be a pre-filled syringe. The above device may be sterilized.

Another aspect provides a kit including the pre-filled syringe described above. The kit may include an instruction manual including information on administration of the composition.

Another aspect provides a method of filling tissue of a subject or hydrating skin of a subject, including administering to the subject a therapeutically effective amount of the composition described above. The method may be for augmentation, restoration, or reinforcement of a subject's tissue, for filling a body cavity, or for hydrating the skin. In this context, augmentation, restoration, or reinforcement of tissue or filling of a body cavity may be a result of increased volume caused by the injected composition, or a secondary effect associated with skin hydration. The component forming the composition may be evenly spread from the injection site to the surrounding area without experiencing a lump phenomenon. In the method, the "composition" and "subject" are as described above. The administration may be into the skin, such as dermis, or into a joint cavity. In the method, the administration may be performed into the skin, for example, into the dermis, using a syringe, such as a pre-filled syringe. In the method, the administration may involve injecting 0.1 ml to 50 ml, 0.5 ml to 30 ml, 0.5 ml to 20 ml, 0.5 ml to 15 ml, or 0.8 ml to 12 ml per dose. In the method, the administration of the composition may be performed once every period of 3 months or more, 4 months or more, 5 months or more, 6 months or more, 12 months or more, or 18 months or more. For example, the administration may be performed once or multiple times at an interval of 28 days or more, 29 days or more, 30 days or more, 31 days or more, 32 days or more, 33 days or more, 34 days or more, 35 days or more, 36 days or more, 37 days or more, 38 days or more, 39 days or more, 40 days or more, 41 days or more, 42 days or more, 43 days or more, 44 days or more, 45 days or more, 46 days or more, 47 days or more, 48 days or more, 49 days or more, 50 days or more, 51 days or more, 52 days or more, 53 days or more, 54 days or more, 55 days or more, 56 days or more, 57 days or more, 58 days or more, 59 days or more, 60 days or more, 61 days or more, 62 days or more, 63 days or more, 64 days or more, 65 days or more, 66 days or more, 67 days or more, 68 days or more, 69 days or more, 70 days or more, 71 days or more, 72 days or more, 73 days or more, 74 days or more, 75 days or more, 76 days or more, 77 days or more, 78 days or more, 79 days or more, 80 days or more, 81 days or more, 82 days or more, 83 days or more, 84 days or more, 85 days or more, 86 days or more, 87 days or more, 88 days or more, 89 days or more, 90 days or more, 91 days or more, 92 days or more, 93 days or more, 94 days or more, 95 days or more, 96 days or more, 97 days or more, 98 days or more, 99 days or more, 100 days or more, 101 days or more, 102 days or more, 103 days or more, 104 days or more, 105 days or more, 106 days or more, 107 days or more, 108 days or more, 109 days or more, 110 days or more, 111 days or more, 112 days or more, 180 days or more, 28 days to 84 days, 29 days to 84 days, 30 days to 84 days, 35 days to 84 days, 40 days to 84 days, 45 days to 84 days, 50 days to 84 days, 54 days to 84 days, 55 days to 84 days, 60 days

to 84 days, 28 days to 112 days, 29 days to 112 days, 30 days to 112 days, 35 days to 112 days, 40 days to 112 days, 45 days to 112 days, 50 days to 112 days, 54 days to 112 days, 55 days to 112 days, 60 days to 112 days, 70 days to 112 days, 80 days to 112 days, 90 days to 112 days, 100 days to 112 days, or 105 days to 112 days, 28 days to 180 days, 56 days to 180 days, 56 days to 180 days, or 112 days to 180 days. The administration may be performed to a local region requiring tissue filling or skin hydration. The administration may be to the skin, face, breast, joint, or a combination thereof.

Advantageous Effects

The claimed cross-linked hyaluronic acid gel according to an aspect has good spreadability, so when injected into the body, lump phenomenon does not occur, and it has good stability, allowing it to be maintained for a long time.
A composition for filling or skin hydration including the cross-linked hyaluronic acid gel according to another aspect may be used for filling or skin hydration.
By a method of filling the tissue of a subject according to another aspect, the tissue of the subject may be filled efficiently.
By a method of hydrating the skin of a subject according to another aspect, the skin of the subject may be efficiently hydrated, and the skin moisturizing environment may be improved.

Description of Drawings

FIG. 1 is a diagram showing the results of measuring the volume of an injection site after injecting a cross-linked hyaluronic acid composition into the back of a mouse.
FIGS. 2A, 2B and 2C are photographs showing visual observation of volume changes at the injection site after injecting a cross-linked hyaluronic acid gel into a mouse.
FIGS. 3A and 3B are stained photographs of cross-sections of the central and lateral areas of the injection site 28 days after injection of a cross-linked hyaluronic acid gel into a mouse.
FIGS. 4A and 4B are photographs of stained cross-sections of the central and lateral areas of the injection site 56 days after injection of a cross-linked hyaluronic acid gel into a mouse.
FIGS. 5A and 5B are photographs of stained cross-sections of the central and lateral areas of the injection site 84 days after injection of a cross-linked hyaluronic acid gel into a mouse.
FIGS. 6A and 6B are photographs of stained cross-sections of the central and lateral areas of the injection site 112 days after injection of a cross-linked hyaluronic acid gel into a mouse.
FIGS. 7A and 7B are photographs of stained cross-sections of the central and lateral areas of the injection site 180 days after injection of a cross-linked hyaluronic acid gel into a mouse.

Best Mode

Hereinafter, the present invention will be described in more detail through examples. However, these examples are intended to exemplify the present invention and the scope of the present invention is not limited to these examples.

**Materials and Methods**

In the examples below, the following materials and methods were used.

**1. Preparation of Control and Cross-linked Hyaluronic Acid Gels**

**(1) Control Cross-linked Hyaluronic Acid Gel**

As controls, the following commercially available cross-linked hyaluronic acid gel products were used: Belotero soft with lidocaine (Merz Aesthetics), Juvederm Volite (Allergan), and Neuramis Light Lidocaine (Medytox). Belotero Soft with Lidocaine (hereinafter referred to as "BSL") has the property of spreading well from the injection site to the surrounding tissue without lifting or lump phenomenon upon in vivo injection. BSL has remarkably low G', G" and elastic percentage, so it spreads well from the injection site into the surrounding tissues, and does not cause swelling, lifting, or lump formation at the injection site in the early stage of injection, but the duration is short.
Juvederm Volite (hereinafter referred to as "JV") and Neuramis Light Lidocaine (hereinafter referred to as "NLL") are products generally used to correct fine wrinkles. They are products that tend to cause swelling and lifting at the injection site in the early stage of injection, which increases the volume of the injection site. Both products are known to increase in

volume after injection.

**(2) Preparation of Cross-linked Hyaluronic Acid Gel in the Test group**

First, NaOH was dissolved in water to prepare a 0.25N NaOH solution. Into the prepared 0.25N NaOH solution, 7.5 g of sodium hyaluronate (IV 2.0 to 3.0) was added and mixed to make a 15.0 % (w/w) mixture, and the mixture was stirred until fully dissolved. Here, IV refers to intrinsic viscosity. To the above solution, 0.189 g of butanediol diglycidyl ether (BDDE) (Sigma-Aldrich), corresponding to 5 mol% relative to 1 mol of hyaluronate monomer, was added and further stirred to ensure thorough mixing. The mixture was then taken and incubated at 40 °C for 18 hours to carry out the crosslinking reaction and produce a cross-linked hyaluronic acid gel.

Next, the obtained cross-linked hyaluronic acid gel was subjected to dialysis using a NaCl aqueous solution and PBS (pH 7.0) as the dialysis solution to remove unreacted cross-linking agents. After completing dialysis, PBS (1×) was used to adjust the concentration, taking into account the loss rate from the initial weight of sodium hyaluronate, such that the final concentration of sodium hyaluronate was 20 mg/mL. At this time, lidocaine hydrochloride monohydrate was also adjusted to a concentration of 3 mg/mL. The resulting cross-linked hyaluronic acid gel contained 20 mg/mL of cross-linked hyaluronic acid gel and 3 mg/mL of lidocaine hydrochloride monohydrate in PBS. The obtained cross-linked hyaluronic acid gel was then pulverized as described below and used as samples for Test Group 1 to 2.

The prepared cross-linked hyaluronic acid gel was placed into the container of a mixer (Retsch GM-200) and pulverized at 4000 rpm for 3 minutes. As a result, a composition containing 20 mg/mL of the pulverized cross-linked hyaluronic acid gel and 3 mg/mL of lidocaine was prepared, and this is referred to as Test Group 1 or TA1.

A cross-linked hyaluronic acid gel prepared in the same manner as in Test Group 1 was placed into the container of a mixer (Retsch GM-200) and pulverized at 4000 rpm for 10 minutes. As a result, a composition containing 20 mg/mL of pulverized cross-linked hyaluronic acid gel and 3 mg/mL of lidocaine was prepared. This composition, in which the cross-linked hyaluronic acid gel was pulverized into smaller particle sizes compared to Test Group 1, is referred to hereinafter as Test Group 2 or TA2.

Subsequently, 1 mL of each of the cross-linked hyaluronic acid gel and lidocaine-containing compositions from Test Group 1 (TA1) and Test Group 2 (TA2) was filled into glass syringes and subjected to high-temperature steam sterilization.

The average particle size of the cross-linked hyaluronic acid gel in the compositions of Test Groups 1 and 2 was 111.2 μm and 42.3 μm, respectively. The particle size was measured according to the method described below.

Additionally, cross-linked hyaluronic acid gels for Test Groups 3 and 4 were prepared as follows:

NaOH was dissolved in water to prepare a 0.25N NaOH solution. Into the prepared 0.25N NaOH solution, 391 g of sodium hyaluronate (IV 2.0 to 3.0) was added and mixed to make a 15% (w/w) mixture, and the mixture was stirred until fully dissolved. Here, IV refers to intrinsic viscosity. To the solution, 9.8 g of butanediol diglycidyl ether (BDDE) (Sigma-Aldrich), corresponding to 5 mol% relative to 1 mol of hyaluronate monomer, was added and further stirred to ensure thorough mixing. The mixture was then taken and incubated at 40 °C for 18 hours to carry out the crosslinking reaction and produce a cross-linked hyaluronic acid gel.

Next, the obtained cross-linked hyaluronic acid gel was sealed in a dialysis membrane and subjected to dialysis using NaCl aqueous solution and PBS (pH 7.0) as the dialysis solutions in order to remove unreacted cross-linking agents. After completion of dialysis, concentration adjustment was performed using 1× PBS so that the final concentration of sodium hyaluronate, considering the loss rate from the initially added amount, would be 20 mg/mL. At this time, lidocaine hydrochloride monohydrate was also adjusted to a concentration of 3 mg/mL. The resulting cross-linked hyaluronic acid gel contained 20 mg/mL of cross-linked hyaluronic acid gel and 3 mg/mL of lidocaine hydrochloride monohydrate in PBS. The obtained gel was then pulverized using a grinder (MP-50, IKA) at 6,500 rpm for 60 minutes and 90 minutes, respectively, to obtain Test Groups 3 and 4.

Subsequently, 1 mL of each of the cross-linked hyaluronic acid gel and lidocaine-containing compositions from Test Groups 3 and 4 was filled into glass syringes and subjected to high-temperature steam sterilization.

The average particle size of the cross-linked hyaluronic acid gel in the compositions of Test Groups 3 and 4 was 61.3 μm and 46.4 μm, respectively. Particle size was measured after sterilization according to the method described below.

**2. Methods**

**(2.1) Method for Measuring Elastic Modulus (G') and Viscous Modulus (G")**

Analysis conditions of DHR-2 rheometer device (TA instruments)

(1) Frequency: 0.1 Hz

(2) Temperature: 25 °C
(4) Strain: 1.5 %
(5) Measuring geometry: 40 mm plate
(6) Measuring gap: 1.0 mm

**(2.2) Method for Measuring Elasticity Percentage**

Elasticity (%) is calculated using the measured values of elastic modulus and viscous modulus obtained by the method in Section 2.1, according to the following formula:

Elasticity % = Elastic modulus (G')/(Elastic modulus (G') + Viscous modulus (G")) × 100

**(2.3) Method for Measuring Degradation Time**

Degradation time is measured using the rheometer described in Section 2.1 under the following conditions:

(1) hyaluronidase from *Streptomyces* ≥300 units, Sigma-Aldrich, CAS No. 37259-53-3), final test solution concentration: 3.27 unit/ml)
(2) Frequency: 0.1 Hz
(3) Temperature: 37 °C
(4) Strain: 1.5%
(5) Measurement geometry: 40 mm plate
(6) Measurement gap: 1.0 mm
(7) Determination of degradation time: The time at which the elastic modulus reaches a value closest to 50% of its initial measurement is recorded as the degradation 50 time.

**(2.4) Particle Size Measurement Method**

Particle size was analyzed using the Particle Size Analyzer S3500 from Microtrac under the following conditions:

(1) Sample preparation: 100 μL of gel was mixed with 900 μL of purified water to disperse the particles.
(2) Refractive Index: Hyaluronic acid gel (HA Gel): 1.37, water: 1.333
(3) Flow rate: 70 %
(4) Measurement value: 50 %
(5) The average value of three measurements was used as the particle size value for each sample.

**Example 1: Evaluation of Characteristics of Cross-linked Hyaluronic Acid Gels**

The physical properties of the cross-linked hyaluronic acid gels from the control and test groups described above were evaluated. Each material from the control and test groups was injected into mice, and the degree of spread, presence or absence of lump formation, and the degree to which the material remained over time without degradation (i.e., stability) were assessed.

**1. Physical Properties of Cross-linked Hyaluronic Acid Gels**

Table 1 shows the physical properties of the cross-linked hyaluronic acid gels from the control and test groups.

[Table 1]

| Materials | G' (Pa) | G" (Pa) | Elasticity % | Cross-link Rate (%) | Average Particle Size (μm) | Degradation 50 (minutes) |
|---|---|---|---|---|---|---|
| Test Group 1 | 3.18 | 3.63 | 46.7 | 1.33 | 111.2 | 256 |
| Test Group 2 | 0.24 | 0.81 | 23.0 | 1.45 | 42.3 | 241 |
| Test Group 3 | 0.236 | 0.834 | 21.5 | 2.33 | 61.3 | 96 |
| Test Group 4 | 0.100 | 0.577 | 14.8 | 2.33 | 46.4 | 216 |
| Control Group 1 | 3.97 | 5.25 | 43.06 | 1.31 | 31.0 | 41 |

(continued)

| Materials | G' (Pa) | G" (Pa) | Elasticity % | Cross-link Rate (%) | Average Particle Size (μm) | Degradation 50 (minutes) |
|---|---|---|---|---|---|---|
| Control Group 2 | 22.10 | 12.29 | 64.27 | - | 333.6 | 71 |
| Control Group 3 | 102.70 | 30.40 | 77.16 | 1.77 | 353.9 | 331 |

In Table 1, Control Groups 1, 2, and 3 represent Belotero soft with lidocaine (Merz Aesthetics) (BSL), Neuramis Light Lidocaine (Meditox) (NLL), and Juvederm Volite (Allergan) (JV), respectively. **2. In Vivo spreadability Evaluation of Cross-linked Hyaluronic Acid Gels**

Next, the cross-linked hyaluronic acid gels from the above-mentioned control and test groups were injected into mice, and the volume changes at the injection sites were observed to assess the degree of lumping and spreading.

Hairless mice were used for the experiment. The mice were 6-week-old females with a body weight of 20 $\pm$ 3 g. After being acclimated in the breeding room for one week, 0.1 mL of each gel was administered to the injection site in the 7th week using a syringe. The observation period for the injected cross-linked hyaluronic acid gels was 6 months in total. Measurements were taken immediately after injection (day 0), on days 3, 7, and 14, and at 1, 2, 3, 4, and 6 months after injection using the Primos lite system (PRIMOS 45 $\times$ 33 mm, Canfield Scientific Inc., NJ, USA) to measure the height of the injection site, the maximum length of the injected material, and its volume. Each material was injected once at one site per mouse, with a total of 17 mice used for the study.

FIG. 1 is a diagram showing the results of measuring the volume at the injection site after injecting a cross-linked hyaluronic acid gel composition into the back of a mouse. As shown in FIG. 1, the cross-linked hyaluronic acid gels of Test Group 2 (TA2) and Control Group 1 (BSL) did not exhibit any additional volume increase starting from day 3, the first day of measurement post-injection, when the volume of the initially injected material was set as 100%. In other words, the cross-linked hyaluronic acid gels of Test Group 2 (TA2) and Control Group 1 (BSL) showed a complete reduction of the initial injection volume to 0%, and this state was maintained until the end of the 6-month observation period. This indicates that the gel spread well at the injection site without swelling, lifting, or lump formation in the early phase after injection.

FIGS. 2A, 2B, and 2C are photographs visually observing the volume change at the injection site after injecting the cross-linked hyaluronic acid gel into mice. FIGS. 2A, 2B, and 2C correspond to the observations on days 0, 3, and 7, respectively, after injection. As shown in FIG. 2A, the injection site was swollen in all mice on day 0 after injection. As shown in FIG. 2B, on day 3 after injection, the injection site volume was greatly reduced in Test Groups 1, 2, and Control Group 1, whereas in Control Groups 2 and 3, the injection site remained swollen, and irregular lumps were observed. The injection site volumes were: Control Group 2 and Control Group 3 >>> Test Group 1 > Test Group 2 and Control Group 1.

As shown in FIG. 2C, on day 7 after injection, no swelling or irregular lump formation was observed at the injection site in Test Group 2 and Control Group 1. However, in Test Group 1, Control Group 2, and Control Group 3, the injection sites remained swollen, and uneven lumps were observed. In the figure, the green area indicates the base mat.

Based on these visual observations, it was confirmed that the cross-linked hyaluronic acid gel of Test Group 2 exhibited the same behavior as the gel of Control Group 1, with no swelling or lump formation observed around day 3, indicating that the material was well dispersed into the surrounding tissue.

**3. Evaluation of In Vivo Stability of Cross-linked Hyaluronic Acid Gel**

Next, to evaluate the degradation resistance, i.e., the stability, of the materials, the above-mentioned control and test group cross-linked hyaluronic acid gels were injected into mice, and the extent of remaining material at the injection site over time was assessed. The injection site, formulation, and method were the same as those described in Section 2.

At the designated days after administration, cross-sectional tissue samples of approximately 1 $\times$ 1 cm were collected from the central (median) region of the injection site and the lateral (sagittal) region located 4 mm away from the center. Paraffin embedding was performed on these tissue samples. By examining both the central and lateral sites, the degree to which the cross-linked hyaluronic acid gel had dispersed from the injection site into the surrounding tissue was evaluated. The paraffin-embedded tissue sections were stained using Alcian blue.

FIGS. 3A and 3B are stained images of the central and lateral cross-sections of the injection site 28 days after injection of the cross-linked hyaluronic acid gel into mice. FIGS. 3A and 3B respectively correspond to Test Group 1, Test Group 2, Control Group 2, Control Group 3, and Control Group 1.

FIGS. 4A and 4B are stained images of the central and lateral cross-sections of the injection site 56 days after injection of the cross-linked hyaluronic acid gel into mice. FIGS. 4A and 4B respectively correspond to Test Group 1, Test Group 2, Control Group 2, Control Group 3, and Control Group 1.

FIGS. 5A and 5B are stained images of the central and lateral cross-sections of the injection site 84 days after injection of the cross-linked hyaluronic acid gel into mice. FIGS. 5A and 5B correspond to Test Group 1, Test Group 2,

Control Group 2, Control Group 3, and Control Group 1, respectively.

FIGS. 6A and 6B are stained images of the central and lateral cross-sections of the injection site 112 days after injection of the cross-linked hyaluronic acid gel into mice. FIGS. 6A and 6B correspond to Test Group 1, Test Group 2, Control Group 2, Control Group 3, and Control Group 1, respectively.

FIGS. 7A and 7B are stained images of the central and lateral cross-sections of the injection site 180 days after injection of the cross-linked hyaluronic acid gel into mice. FIGS. 7A and 7B correspond to Test Group 1, Test Group 2, Control Group 2, Control Group 3, and Control Group 1, respectively.

In FIGS. 3A and 3B, FIGS. 4A and 4B, FIGS. 5A and 5B, FIGS. 6A and 6B, and FIGS. 7A and 7B, blue indicates the cross-linked hyaluronic acid gel and red indicates the dermis. As shown in the above figures, up to day 84 post-injection, the cross-linked hyaluronic acid gels of Test Group 1, Control Group 2, and Control Group 3 were scarcely dispersed laterally and remained aggregated at the injection site. The degree of aggregation was in the order: Control Group 3 > Control Group 2 > Test Group 1. In addition, lump formation was observed on the dermal surface in Test Group 1, Control Group 2, and Control Group 3.

Test Group 2 and Control Group 1 showed relatively even lateral dispersion without aggregation in the central region (injection site), and no or minimal lumps were observed on the dermal surface. However, on day 28, the staining intensity differed between Test Group 2 and Control Group 1, with Test Group 2 showing stronger staining. This indicates that a greater amount of cross-linked hyaluronic acid gel remained in Test Group 2 than in Control Group 1. Control Group 1 began to degrade gradually from day 28, and by day 56, no filler material remained. This suggests that Control Group 1 (i.e., BSL) exhibits excellent dispensability but lower degradation resistance compared to the gels of the test group.

Tables 2, 3, 4, 5, and 6 present the degree of residual cross-linked hyaluronic acid gel at 28, 56, 84, 112, and 180 days after injection, based on the results shown in FIGS. 3A and 3B, 4A and 4B, 5A and 5B, 6A and 6B, and 7A and 7B, respectively.

[Table 2]

| Gro up | Test Group 1 | | | Test Group 2 | | | Control Group 1 | | | Control Group 2 | | | Control Group 3 | | |
|---|---|---|---|---|---|---|---|---|---|---|---|---|---|---|---|
| Subj ect No. | 1 | 2 | 3 | 1 | 2 | 3 | 1 | 2 | 3 | 1 | 2 | 3 | 1 | 2 | 3 |
| Cen tral | O | O | O | △ | △ | O | X | △ | △ | O | O | O | O | O | O |
| Late ral | X | O | O | △ | △ | X | △ | X | △ | O | X | X | O | O | X |

[Table 3]

| Gro up | Test Group 1 | | | Test Group 2 | | | Control Group 1 | | | Control Group 2 | | | Control Group 3 | | |
|---|---|---|---|---|---|---|---|---|---|---|---|---|---|---|---|
| Subj ect No. | 1 | 2 | 3 | 1 | 2 | 3 | 1 | 2 | 3 | 1 | 2 | 3 | 1 | 2 | 3 |
| Cen tral | O | O | O | △ | O | △ | X | X | X | O | O | O | O | O | O |
| Late ral | △ | △ | △ | △ | X | △ | X | X | X | X | X | △ | X | O | X |

[Table 4]

| Gro up | Test Group 1 | | | Test Group 2 | | | Control Group 1 | | | Control Group 2 | | | Control Group 3 | | |
|---|---|---|---|---|---|---|---|---|---|---|---|---|---|---|---|
| Subj ect No. | 1 | 2 | 3 | 1 | 2 | 3 | 1 | 2 | 3 | 1 | 2 | 3 | 1 | 2 | 3 |
| Cen tral | O | O | O | △ | △ | O | X | X | X | O | O | O | O | O | O |
| Late ral | O | △ | O | O | X | X | X | X | X | X | X | X | X | X | X |

[Table 5]

| Gro up | Test Group 1 | | | Test Group 2 | | | Control Group 1 | | | Control Group 2 | | | Control Group 3 | | |
|---|---|---|---|---|---|---|---|---|---|---|---|---|---|---|---|
| Subj ect No. | 1 | 2 | 3 | 1 | 2 | 3 | 1 | 2 | 3 | 1 | 2 | 3 | 1 | 2 | 3 |
| Cen tral | O | O | O | △ | △ | △ | X | X | X | O | O | O | O | O | O |
| Late ral | O | O | X | X | X | X | X | X | X | △ | △ | X | X | X | △ |

【Table 6】

| Group | Test Group 1 | | | | Test Group 2 | | | | Control Group 1 | | | | Control Group 2 | | | | Control Group 3 | | | |
|---|---|---|---|---|---|---|---|---|---|---|---|---|---|---|---|---|---|---|---|---|
| Subject No. | 1 | 2 | 3 | 4 | 1 | 2 | 3 | 4 | 1 | 2 | 3 | 4 | 1 | 2 | 3 | 4 | 1 | 2 | 3 | 4 |
| Central | O | O | O | O | △ | △ | △ | △ | X | X | X | X | O | O | O | △ | O | O | O | O |
| Lateral | △ | X | X | △ | X | X | X | X | X | X | X | X | X | △ | △ | X | X | X | X | X |

In Tables 2, 3, 4, 5, and 6, O indicates that cross-linked hyaluronic acid gel was observed throughout the entire subcutaneous region, △ indicates that cross-linked hyaluronic acid gel was partially observed in the subcutaneous region, and X indicates that cross-linked hyaluronic acid gel was not observed.

**Claims**

1. A cross-linked hyaluronic acid gel having good spreadability and stability upon in vivo injection, wherein the gel has an elastic modulus (G') of 0.01 Pa to 20 Pa,

   a viscous modulus (G") of 0.1 Pa to 20 Pa, and
   an elasticity (%) of 10 % to 40 %.

2. The cross-linked hyaluronic acid gel of claim 1, wherein the spreadability is a volume ratio of 0 to 100 % calculated by the following formula at 0 to 3 days after injection:

   Volume ratio (%) = Volume after injection / Volume at initial injection $\times$ 100.

3. The cross-linked hyaluronic acid gel of claim 1, wherein the gel has a Degradation 50 of 90 minutes to 100 minutes.

4. The cross-linked hyaluronic acid gel of claim 1, wherein the gel has an average particle diameter of 35 $\mu$m to 100 $\mu$m.

5. The cross-linked hyaluronic acid gel of claim 1, wherein the gel is cross-linked with a cross-linking agent having a bifunctional epoxy group.

6. The cross-linked hyaluronic acid gel of claim 4, wherein the cross-linking agent having a bifunctional epoxy group is at least one selected from the group consisting of 1,4-butanediol diglycidyl ether, poly(ethylene glycol) diglycidyl ether, poly(propylene glycol) diglycidyl ether, poly(tetramethylene glycol) diglycidyl ether, polyglycerol polyglycidyl ether, glycerol diglycidyl ether, triethylene diglycidyl ether, trimethylolpropane triglycidyl ether, ethylene diglycidyl ether,

neopentyl glycol diglycidyl ether, and 1,6-hexanediol diglycidyl ether.

**7.** The cross-linked hyaluronic acid gel of claim 1, wherein the gel has a cross-link rate of 0.5 % to 7 %.

**8.** A composition for filling or hydration, comprising the cross-linked hyaluronic acid gel of any one of claims 1 to 6.

**9.** The composition of claim 7, wherein the cross-linked hyaluronic acid gel has a concentration of 10 mg/mL to 30 mg/mL.

**10.** The composition of claim 7, which does not further comprise non-cross-linked hyaluronic acid.

**11.** The composition of claim 7, further comprising a local anesthetic.

**12.** The composition of claim 7, wherein the composition is filled in a syringe.

**13.** The composition of claim 7, which is for use in one or more applications selected from the group consisting of skin hydration, facial plastic surgery, wrinkle improvement, facial contouring, breast plastic surgery, breast augmentation, genital enlargement, urinary incontinence treatment, arthritis treatment, and drug delivery.

**14.** The composition of claim 7, for use in administration to the skin for hydration within tissues.

**15.** The composition of claim 1, further comprising a pharmacologically active material or a material having a biological function.

**16.** The composition of claim 15, wherein the pharmacologically active material or the material having a biological function is at least one material selected from the group consisting of anesthetics, collagen, chitosan, alginate, gelatin, polynucleotide, polydeoxyribonucleotide, silk fibroin, elastin, tropoelastin, carrageenan, gamma-polyglutamic acid, sulfated hyaluronic acid, chondroitin sulfate, fucoidan, heparin, heparan sulfate, pentosan polysulfate, pullulan, keratin, polyethylene glycol, polylactic acid, polyglycolic acid, polylactic-co-glycolic acid, polycaprolactone, poloxamer, polyphosphazene, and polydioxanone.

# FIG. 1

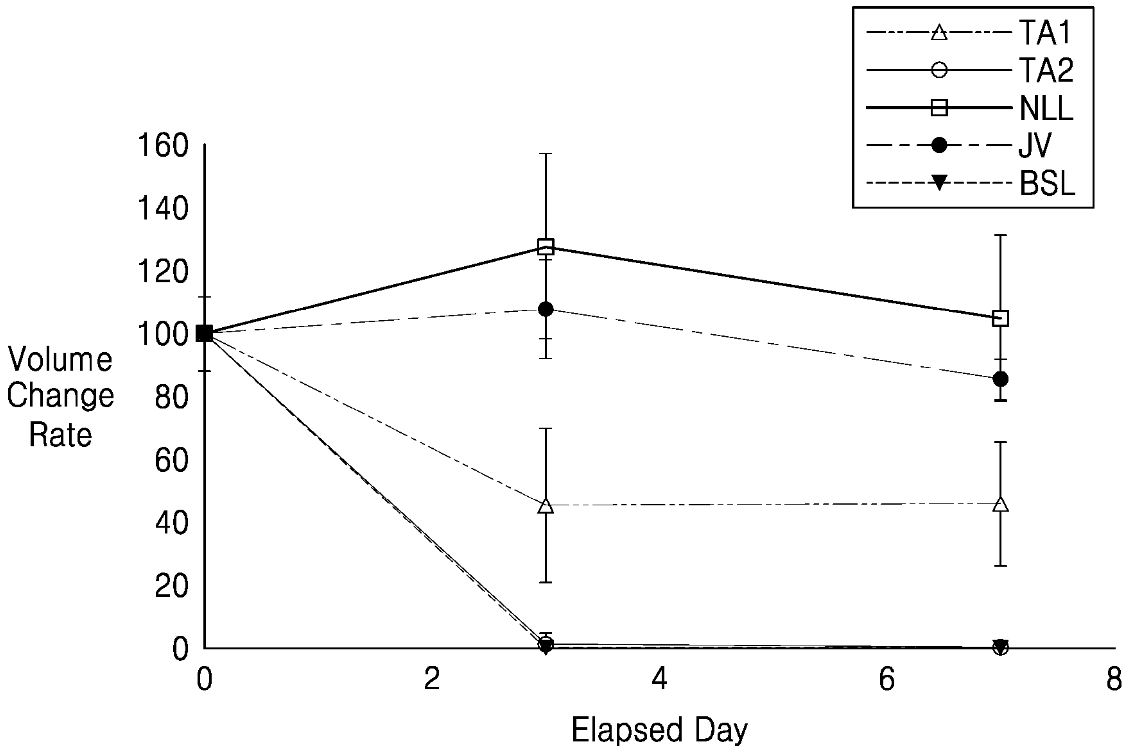
TA1
TA2
NLL
JV
BSL
160
140
120
100
80
60
40
20
0
Volume
Change
Rate
0
2
4
6
8
Elapsed Day

FIG. 2A

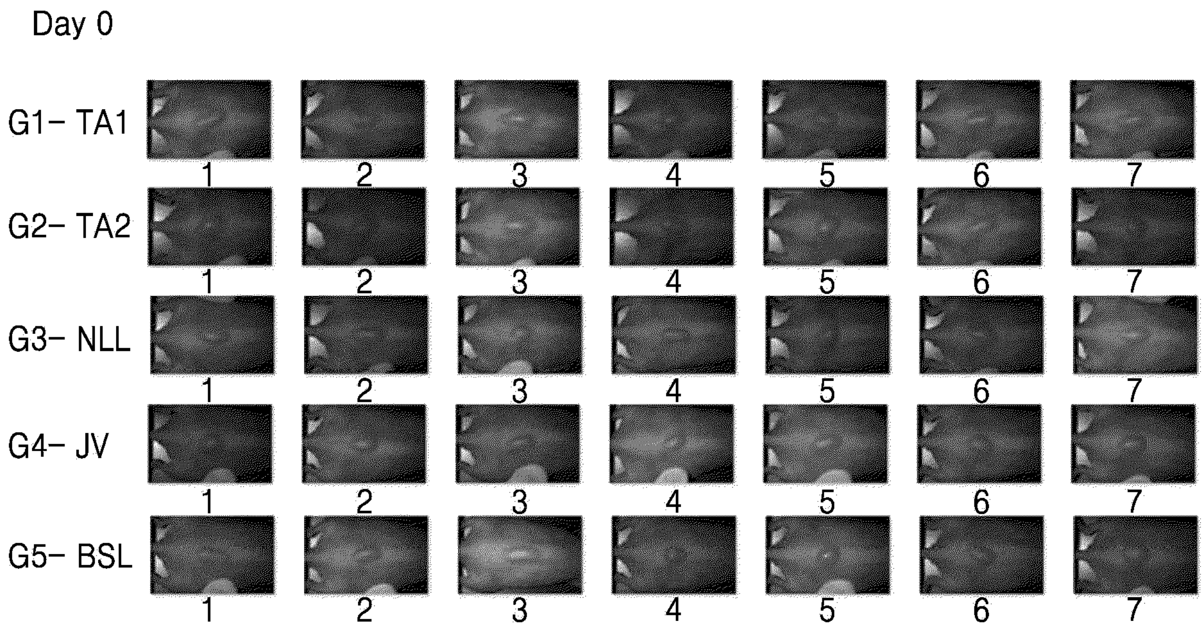
Day 0
G1- TA1
1
2
3
4
5
6
7
G2- TA2
1
2
3
4
5
6
7
G3- NLL
1
2
3
4
5
6
7
G4- JV
1
2
3
4
5
6
7
G5- BSL
1
2
3
4
5
6
7

FIG. 2B

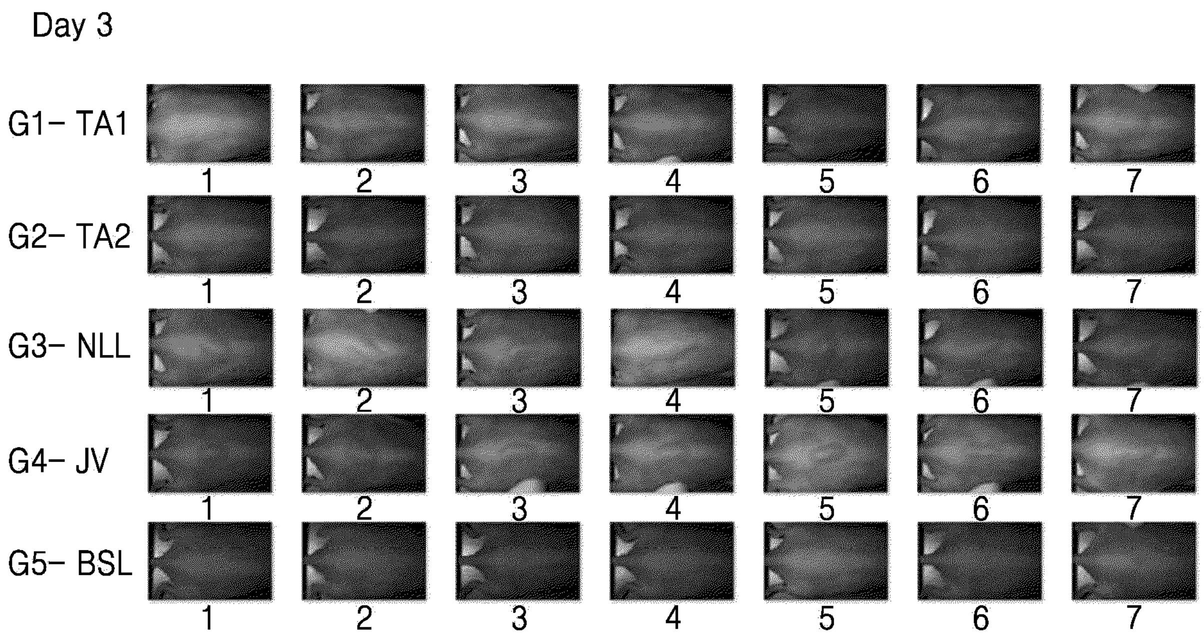
Day 3
G1- TA1
1
2
3
4
5
6
7
G2- TA2
1
2
3
4
5
6
7
G3- NLL
1
2
3
4
5
6
7
G4- JV
1
2
3
4
5
6
7
G5- BSL
1
2
3
4
5
6
7

FIG. 2C

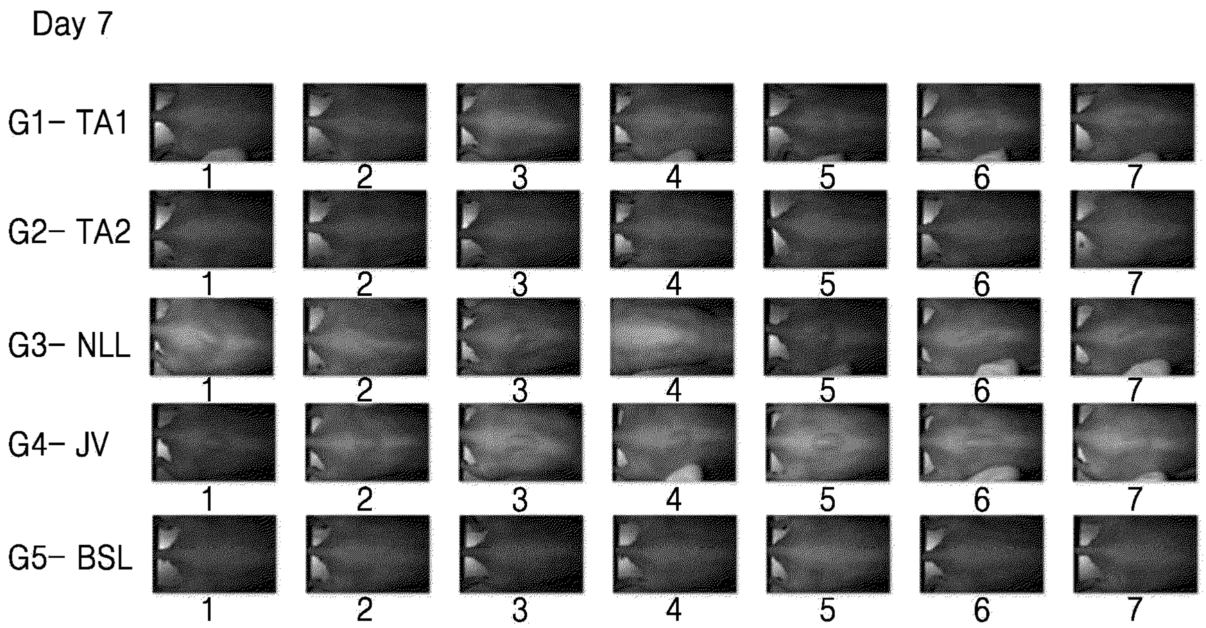

FIG. 3A

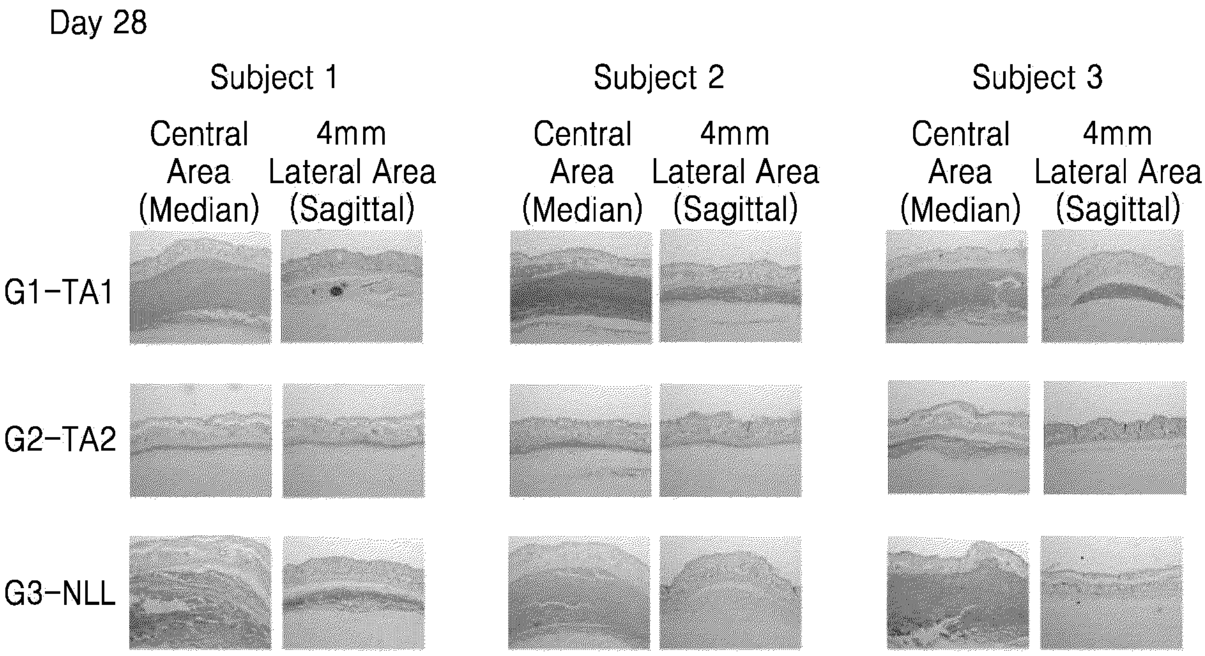
Day 28
Subject 1
Subject 2
Subject 3
Central Area (Median)
4mm Lateral Area (Sagittal)
G1-TA1
G2-TA2
G3-NLL

FIG. 3B

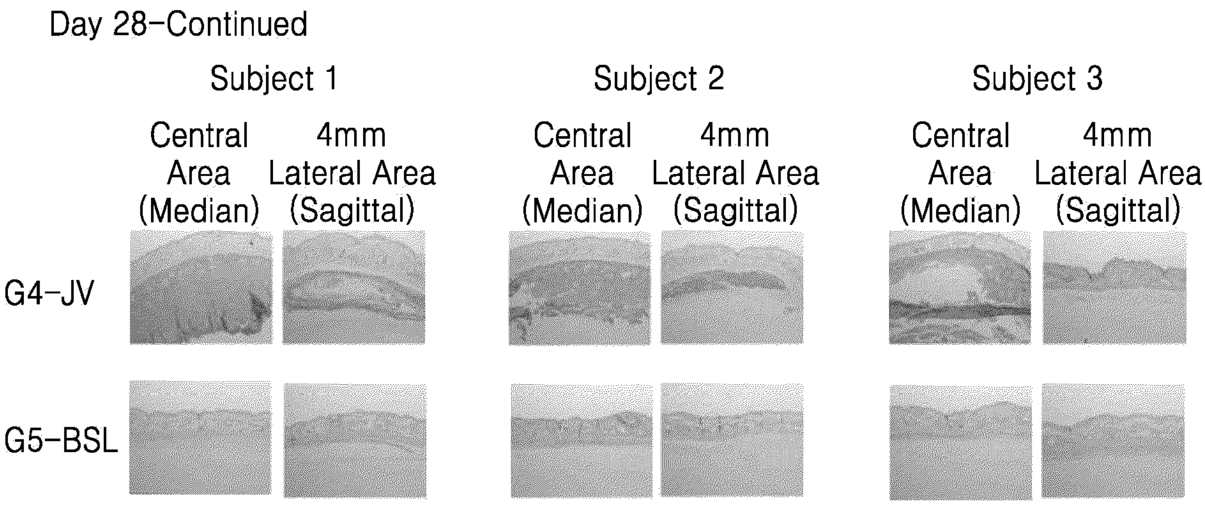
Day 28-Continued
Subject 1
Subject 2
Subject 3
Central Area (Median)
4mm Lateral Area (Sagittal)
G4-JV
G5-BSL

FIG. 4A

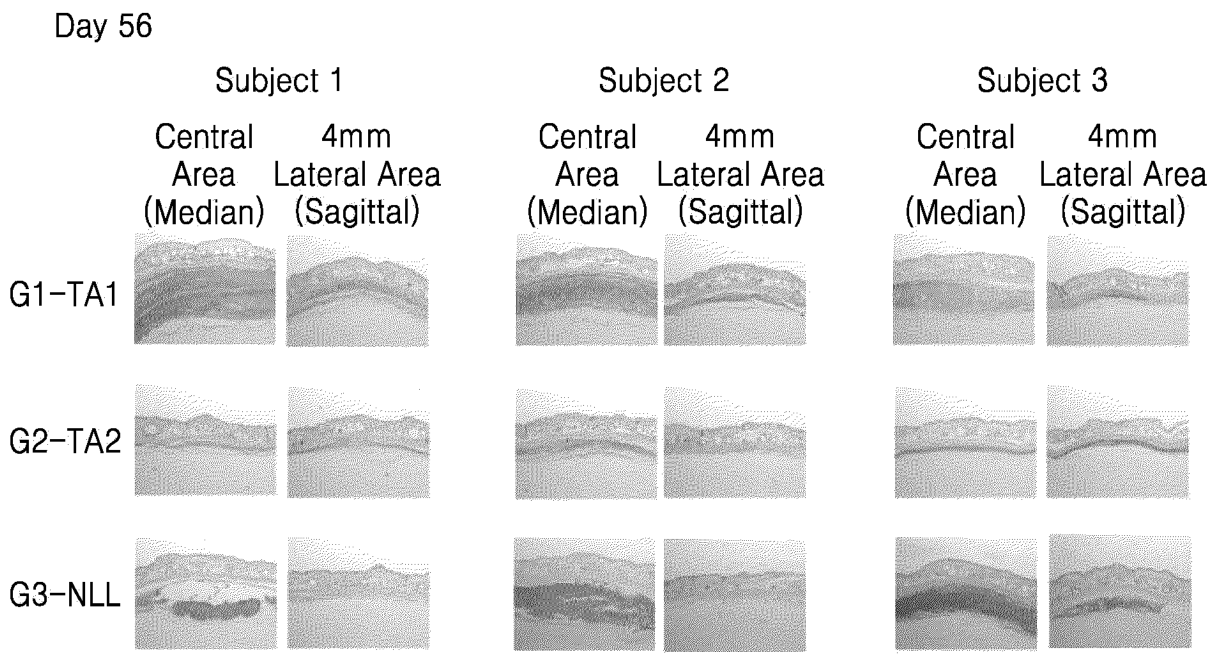
Day 56
Subject 1
Subject 2
Subject 3
Central Area (Median)
4mm Lateral Area (Sagittal)
Central Area (Median)
4mm Lateral Area (Sagittal)
Central Area (Median)
4mm Lateral Area (Sagittal)
G1-TA1
G2-TA2
G3-NLL

FIG. 4B

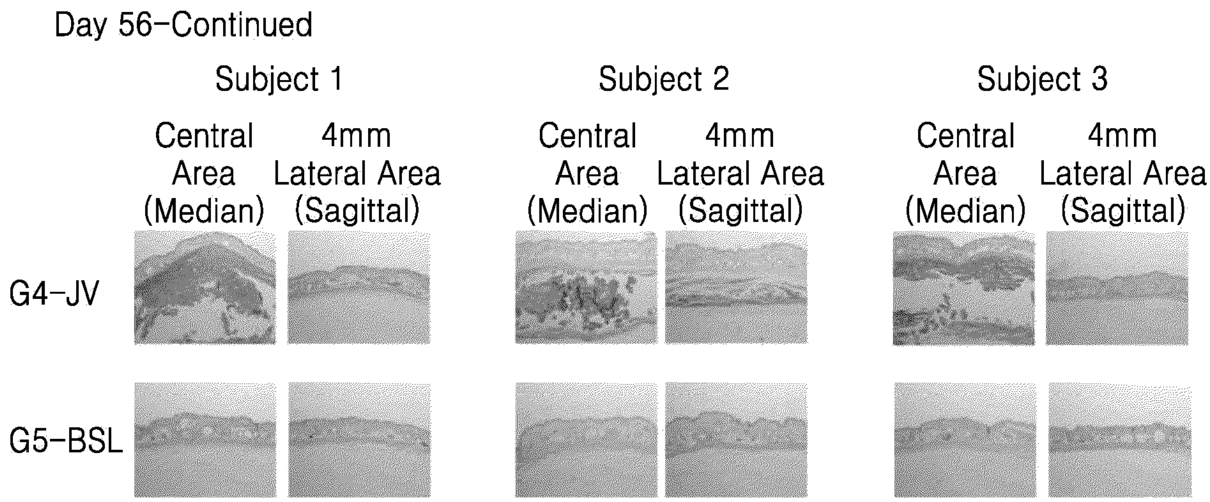
Day 56-Continued
Subject 1
Subject 2
Subject 3
Central Area (Median)
4mm Lateral Area (Sagittal)
Central Area (Median)
4mm Lateral Area (Sagittal)
Central Area (Median)
4mm Lateral Area (Sagittal)
G4-JV
G5-BSL

FIG. 5A

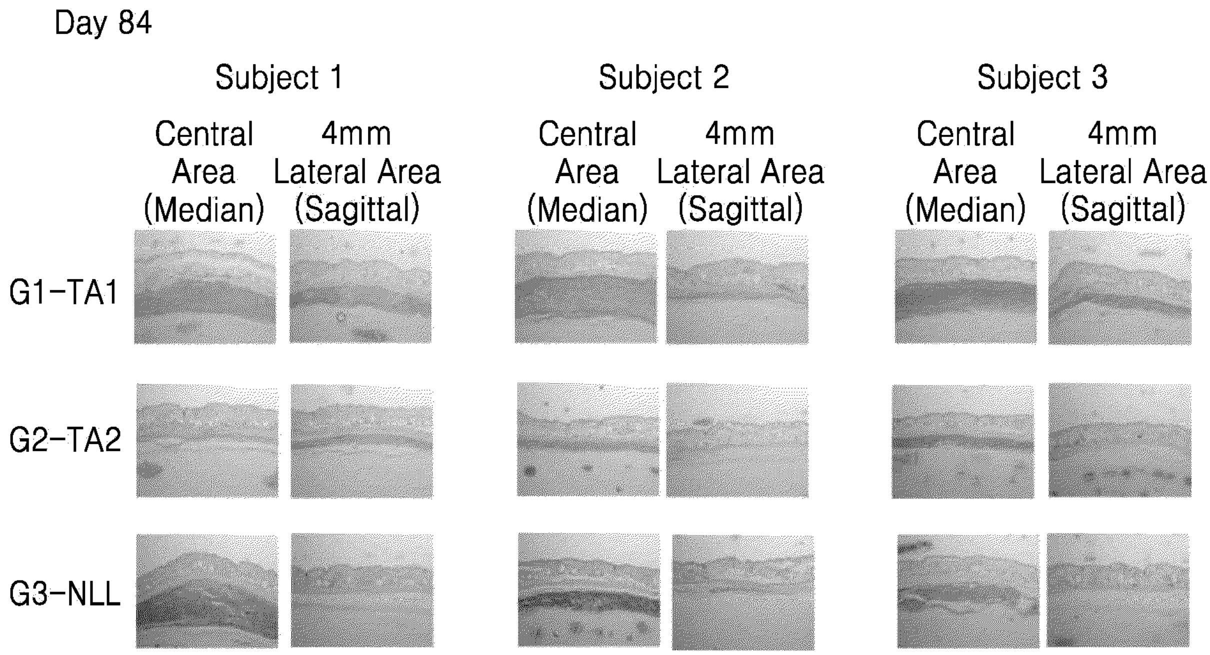
Day 84
Subject 1
Subject 2
Subject 3
Central Area (Median)
4mm Lateral Area (Sagittal)
G1-TA1
G2-TA2
G3-NLL

FIG. 5B

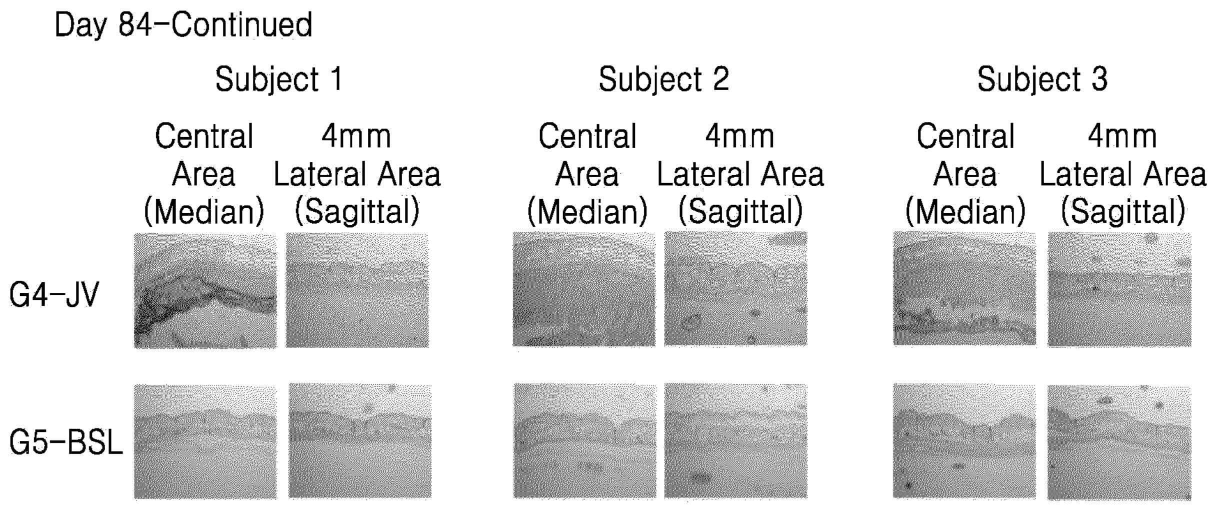
Day 84-Continued
Subject 1
Subject 2
Subject 3
Central Area (Median)
4mm Lateral Area (Sagittal)
G4-JV
G5-BSL

# FIG. 6A

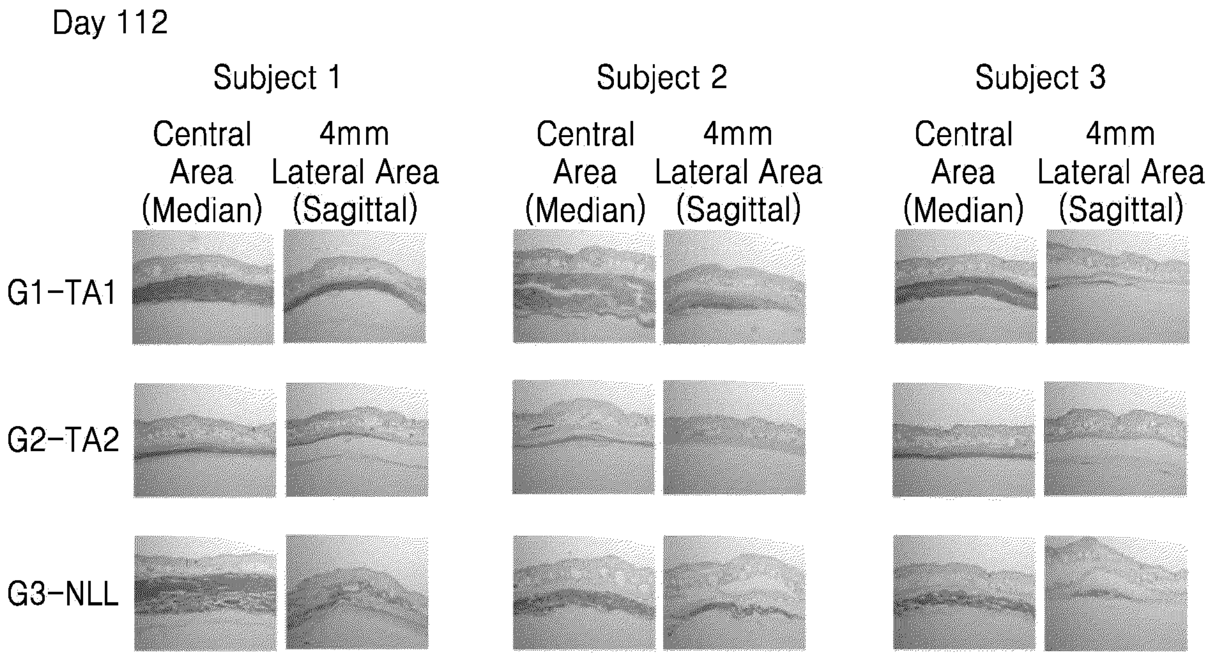
Day 112
Subject 1
Subject 2
Subject 3
Central Area (Median)
4mm Lateral Area (Sagittal)
Central Area (Median)
4mm Lateral Area (Sagittal)
Central Area (Median)
4mm Lateral Area (Sagittal)
G1-TA1
G2-TA2
G3-NLL

# FIG. 6B

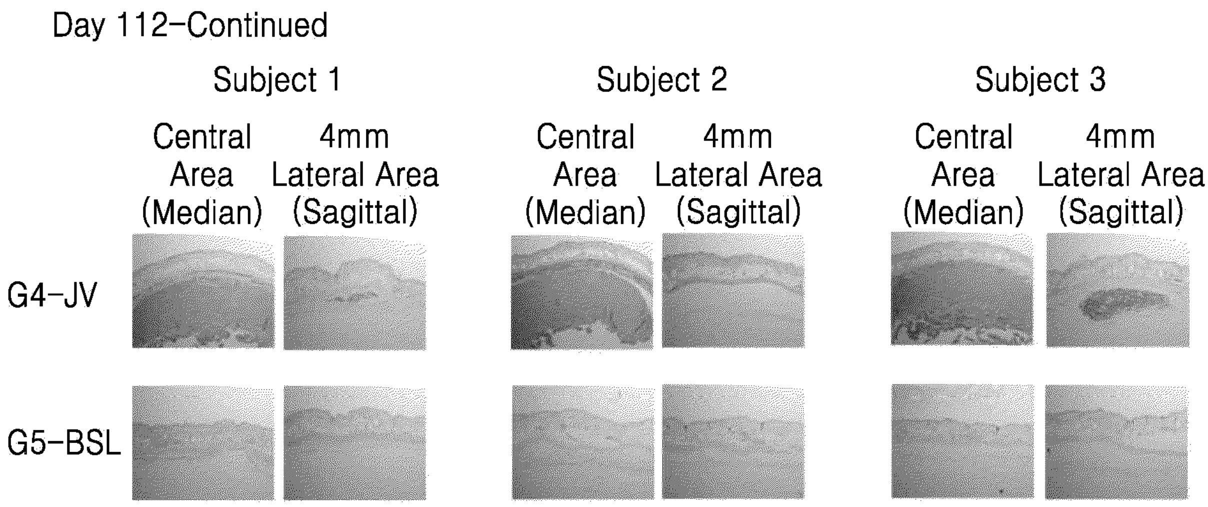
Day 112-Continued
Subject 1
Subject 2
Subject 3
Central Area (Median)
4mm Lateral Area (Sagittal)
Central Area (Median)
4mm Lateral Area (Sagittal)
Central Area (Median)
4mm Lateral Area (Sagittal)
G4-JV
G5-BSL

FIG. 7A

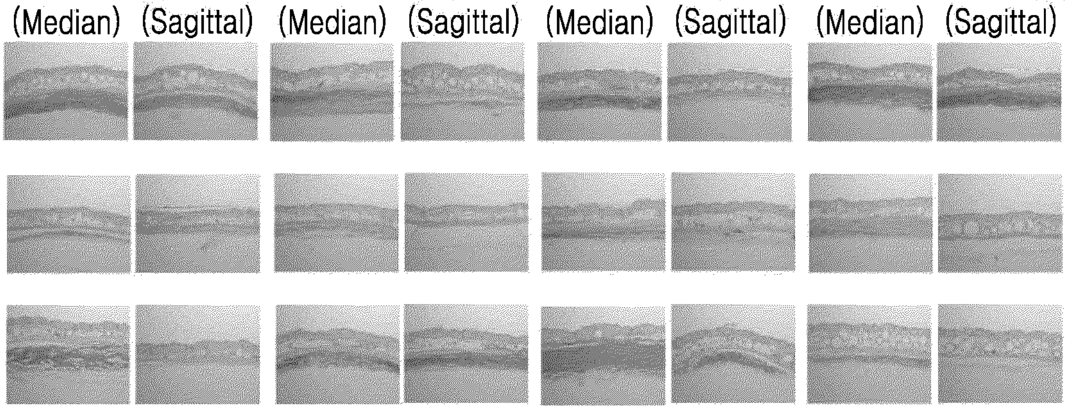
Day 180
Subject 1
Subject 2
Subject 3
Subject 4
Central Area (Median)
4mm Lateral Area (Sagittal)
G1-TA1
G2-TA2
G3-NLL

FIG. 7B

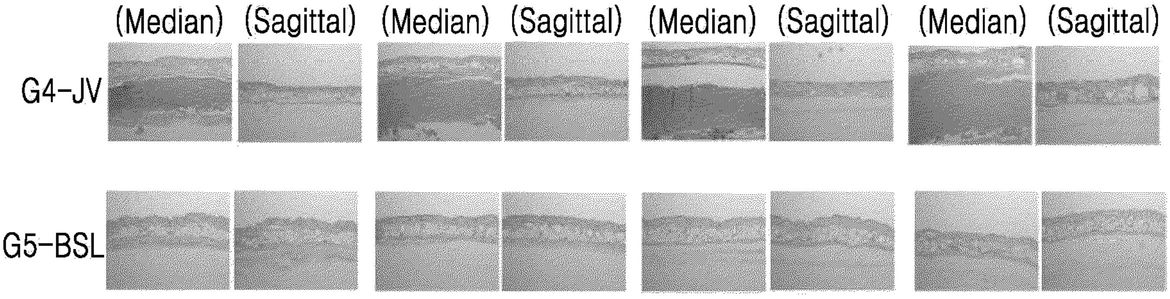
Day 180-Continued
Subject 1
Subject 2
Subject 3
Subject 4
Central Area (Median)
4mm Lateral Area (Sagittal)
G4-JV
G5-BSL

## INTERNATIONAL SEARCH REPORT

International application No.
**PCT/KR2023/019790**

**A. CLASSIFICATION OF SUBJECT MATTER**

**A61L 27/20**(2006.01)i; **A61L 27/52**(2006.01)i; **A61L 27/54**(2006.01)i

According to International Patent Classification (IPC) or to both national classification and IPC

**B. FIELDS SEARCHED**

Minimum documentation searched (classification system followed by classification symbols)

A61L 27/20(2006.01); A61K 31/728(2006.01); A61K 47/36(2006.01); A61K 8/34(2006.01); A61K 8/73(2006.01); A61K 9/08(2006.01); C08B 37/08(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 가교(cross-linking), 1,4-Butanediol diglycidyl ether(BDDE), 히알루론산(hyaluronic acid), 겔(gel), 탄력성(elasticity), 퍼짐성(spreadability), 수화(hydration)

**C. DOCUMENTS CONSIDERED TO BE RELEVANT**

| Category* | Citation of document, with indication, where appropriate, of the relevant passages | Relevant to claim No. |
|---|---|---|
| A | SANTORO, S. et al. Rheological properties of cross-linked hyaluronic acid dermal fillers. Journal of applied biomaterials & biomechanics. 2011, vol. 9, no. 2, pp. 127-136. See abstract, and Tables 1-3. | 1-16 |
| A | KR 10-2173790 B1 (JP CARES) 03 November 2020 (2020-11-03) See paragraph [0073]. | 1-16 |
| A | KR 10-2021-0114284 A (MEDYTOX INC.) 23 September 2021 (2021-09-23) See paragraphs [0028]-[0029], [0033]-[0034], [0038]-[0039] and [0042]-[0046]. | 1-16 |
| A | KR 10-2017-0113660 A (ALLERGAN INDUSTRIE SAS) 12 October 2017 (2017-10-12) See claim 30. | 1-16 |
| A | KR 10-2014-0000206 A (DENKI KAGAKU KOGYO KABUSHIKIKAISHA) 02 January 2014 (2014-01-02) See paragraphs [0086]-[0096]. | 1-16 |

☐ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **08 March 2024** | **08 March 2024** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

**INTERNATIONAL SEARCH REPORT**
**Information on patent family members**

International application No.
**PCT/KR2023/019790**

| Patent document cited in search report | Publication date (day/month/year) | Patent family member(s) | Publication date (day/month/year) |
|---|---|---|---|
| KR 10-2173790 B1 | 03 November 2020 | EP 4112082 A1 | 04 January 2023 |
| | | JP 2023-515181 A | 12 April 2023 |
| | | US 2023-0338353 A1 | 26 October 2023 |
| | | WO 2021-172694 A1 | 02 September 2021 |
| KR 10-2021-0114284 A | 23 September 2021 | BR 112022018028 A2 | 01 November 2022 |
| | | CN 115279798 A | 01 November 2022 |
| | | EP 4119585 A1 | 18 January 2023 |
| | | JP 2023-518171 A | 28 April 2023 |
| | | KR 10-2395184 B1 | 10 May 2022 |
| | | US 2023-0126726 A1 | 27 April 2023 |
| | | WO 2021-182763 A1 | 16 September 2021 |
| KR 10-2017-0113660 A | 12 October 2017 | AU 2016-217963 A1 | 18 August 2016 |
| | | AU 2016-217963 B2 | 07 January 2021 |
| | | AU 2021-202089 A1 | 29 April 2021 |
| | | AU 2021-202089 B2 | 20 July 2023 |
| | | AU 2023-241303 A1 | 26 October 2023 |
| | | BR 112017017043 A2 | 10 April 2018 |
| | | CA 2972561 A1 | 18 August 2016 |
| | | CN 107205911 A | 26 September 2017 |
| | | EP 3256097 A1 | 20 December 2017 |
| | | EP 3256097 B1 | 05 January 2022 |
| | | EP 3988078 A1 | 27 April 2022 |
| | | ES 2910249 T3 | 12 May 2022 |
| | | HK 1244443 A1 | 10 August 2018 |
| | | HK 1248134 A1 | 12 October 2018 |
| | | JP 07425007 B2 | 30 January 2024 |
| | | JP 2018-504448 A | 15 February 2018 |
| | | JP 2021-091694 A | 17 June 2021 |
| | | JP 2024-008963 A | 19 January 2024 |
| | | RU 2017131027 A | 12 March 2019 |
| | | RU 2017131027 A3 | 01 August 2019 |
| | | RU 2020112214 A | 15 May 2020 |
| | | RU 2718873 C2 | 15 April 2020 |
| | | US 11260015 B2 | 01 March 2022 |
| | | US 2020-0345612 A1 | 05 November 2020 |
| | | US 2022-0257495 A1 | 18 August 2022 |
| | | WO 2016-128374 A1 | 18 August 2016 |
| | | WO 2016-128783 A1 | 18 August 2016 |
| KR 10-2014-0000206 A | 02 January 2014 | AU 2011-294295 A1 | 01 March 2012 |
| | | AU 2011-294295 B2 | 26 June 2014 |
| | | CA 2809228 A1 | 01 March 2012 |
| | | CA 2809228 C | 23 January 2018 |
| | | CN 103124558 A | 29 May 2013 |
| | | CN 103124558 B | 24 June 2015 |
| | | EP 2609924 A1 | 03 July 2013 |
| | | JP 05824455 B2 | 25 November 2015 |
| | | KR 10-1834588 B1 | 05 March 2018 |
| | | US 2013-0203697 A1 | 08 August 2013 |
| | | US 9216193 B2 | 22 December 2015 |
| | | WO 2012-026468 A1 | 28 October 2013 |

Form PCT/ISA/210 (patent family annex) (July 2022)

**INTERNATIONAL SEARCH REPORT**
**Information on patent family members**

International application No.
**PCT/KR2023/019790**

| Patent document cited in search report | Publication date (day/month/year) | Patent family member(s) | Publication date (day/month/year) |
|---|---|---|---|
| | | WO 2012-026468 A1 | 01 March 2012 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description

- *CHEMICAL ABSTRACTS*, 37259-53-3